(19) Europäisches Patentamt
European Patent Office
Office européen des brevets

(11) **EP 2 453 798 B1**

(12) **EUROPEAN PATENT SPECIFICATION**

(45) Date of publication and mention
of the grant of the patent:
**27.05.2020 Bulletin 2020/22**

(21) Application number: **10800433.4**

(22) Date of filing: **13.07.2010**

(51) Int Cl.:
**A61B 6/00** *(2006.01)*     **G06T 11/00** *(2006.01)*

(86) International application number:
**PCT/US2010/041871**

(87) International publication number:
**WO 2011/008787 (20.01.2011 Gazette 2011/03)**

(54) **SYSTEM AND METHOD FOR IMAGE RECONSTRUCTION BY USING MULTI-SHEET SURFACE REBINNING**

SYSTEM UND VERFAHREN ZUR BILDREKONSTRUKTION DURCH DIE VERWENDUNG VON MEHRFACHSCHEIBEN-OBERFLÄCHEN-REBINNING

SYSTÈME ET PROCÉDÉ DE RECONSTRUCTION D'IMAGE À L'AIDE D'UN RÉARRANGEMENT À MULTIPLES SURFACES DE FEUILLE

(84) Designated Contracting States:
**AL AT BE BG CH CY CZ DE DK EE ES FI FR GR HR HU IE IS IT LI LT LU LV MC MK MT NL NO PL PT RO SE SI SK SM TR**

(30) Priority: **14.07.2009 US 225257 P**

(43) Date of publication of application:
**23.05.2012 Bulletin 2012/21**

(73) Proprietor: **Rapiscan Systems, Inc.**
**Torrance, CA 90503 (US)**

(72) Inventors:
• **BETCKE, Marta**
**London W5 3TX (GB)**
• **LIONHEART, William Robert, Breckon**
**Whaley Bridge**
**High Peak SK23 7BP (GB)**
• **MORTON, Edward, James**
**Guildford**
**Surrey GU1 2SL (GB)**

(74) Representative: **Barker Brettell LLP**
**Medina Chambers**
**Town Quay**
**Southampton SO14 2AQ (GB)**

(56) References cited:
WO-A1-99/01066          US-A1- 2002 113 215
US-A1- 2004 066 879     US-A1- 2005 123 092
US-A1- 2006 050 842     US-A1- 2008 049 891
US-B1- 6 240 157        US-B1- 6 240 157
US-B1- 6 324 243        US-B1- 6 324 243
US-B1- 6 411 670

**Description**

**FIELD OF THE INVENTION**

The present invention relates to methods for image reconstruction in which radiation is assumed to propagate along straight lines through an attenuating medium. More particularly, the present invention relates to a method of reconstructing images obtained from a cone beam tomography sensor in which detector positioning is restricted, and thus, offset through the presence of the multiple sources.

**BACKGROUND**

In a conventional cone beam X-ray computerized tomography (CB CT) system a source of radiation is placed opposite an array of detectors which are arranged in a manner such that the position of the detector array is fixed relative to the source. The source and detectors are then moved mechanically relative to an object being imaged. In some systems, the object is kept stationary and the source-detector assembly is moved, whereas in others the source and detectors are rotated around the object while the object is translated. Some systems are configured such that the source describes a helical trajectory relative to the object. The rate at which tomographic images can be acquired by such systems is limited by the rate of rotation of the assembly supporting the source and detector array.

In X-ray tomography systems such as in a Real Time Tomography (RTT) system for example, a plurality of X-ray sources are arranged around a circle, however more general arrangements of sources along curves encircling the region of interest are possible. These sources are switched on and off in a sequence in order to obtain the same effect as obtained from a single rotating radiation source. In such systems, a detector cannot be placed opposite a given source as that position is occupied by another source. This renders attenuation along rays that make less than a particular limiting angle to the plane of the sources immeasurable. Such systems maybe termed as "offset detector" systems.

In contrast to the conventional and standard helical cone beam tomography system, for such an "offset detector" system no plane exists in which attenuation along all rays are measured. Hence, a simple two dimensional inverse Radon transform cannot be used to reconstruct an image on that plane. One known method for regaining efficiency of two dimensional reconstructions for such a system is to approximate the line integrals along rays in a plane using integrals along rays that lie close to that plane. A more general method called surface rebinning is to approximate using lines close to a surface.

For a given detector array shape and size and source trajectory, it is possible to calculate an optimal rebinning surface using the fixed point algorithm known in the art. This method can also be used, with some modification, when the extent of the detector is limited, as is in offset systems, or more generally, systems where the detector is not symmetrical in an axial direction with respect to the active source. However in the case of an offset detector approximation with rays close to one surface can result in poor image reconstruction due to absence of rays making an acute angle to the source plane.

Hence, there is need for a method of reconstructing images from a tomographic system in which detectors are not located directly opposite radiation sources.

US 2008/0049891 A1 describes methods for reconstructing x-ray projection data acquired using a multi-source, inverse-geometry computed tomography ("IGCT") scanner. One embodiment of a first method processes an IGCT sinogram by rebinning first in "z" and then in "xy," with feathering applied during the "xy" rebinning. This produces an equivalent of a multi-axial 3rd generation sinogram, which may be further processed using a parallel derivative and/or Hilbert transform. A TOM-window (with feathering) technique and a combined backprojection technique may also be applied to produce a reconstructed volume. An embodiment of a second method processes an IGCT sinogram using a parallel derivative and/or redundancy weighting. The second method may also use signum weighting, TOM-windowing (with feathering), backprojection, and a Hilbert Inversion to produce another reconstructed volume.

US 6,240,157 B1 describes a method in which complete helical cone-beam scanning and non-redundant data acquisition are obtained for three-dimensional tomographic imaging of arbitrary long objects. The minimum sized two-dimensional detector window is bounded by two consecutive turns of the helix. The ray source exposes all object points during a rotation of exactly 180 degrees when seen from the points themselves. Only one-dimensional filtering is employed in the reconstruction. Rebinning to parallel beams, as seen along the axis of rotation, allows for especially simple procedures without any need for pre-weighting or magnification factors. As a special case, the invention is applicable to helical fan-beam scanning with one-dimensional detector arrays.

US 6,411,670 B1 describes a method for producing a tomographic image of an object. The method includes steps of obtaining fan beam projection data of the object from a tomographic scan; rebinning the fan beam projection data into a quantity of parallel projection data points; applying interpolation to the quantity of parallel projection data points to increase the quantity of parallel projection data points; and generating a tomographic image from the increased quantity of parallel projection data points.

## SUMMARY OF THE INVENTION

The present invention provides a tomography system according to claim 1. The system comprises a plurality of X-ray or gamma ray sources and a plurality of detectors, which are offset from one other such that the detectors are not located directly opposite the sources, wherein the plurality of sources lie in a first plane and the plurality of detectors lie in a plurality of planes parallel to the first plane of sources. The tomography system further comprises a controller adapted to process X-rays or gamma rays detected by said plurality of detectors and generate a three dimensional image wherein said controller comprises a plurality of programmatic instructions that, when executed, a) rebin each of said detected X-rays or gamma rays onto a non-flat surface, b) perform a two dimensional reconstruction of said rebinned data on a non-flat surface, and c) generate said three dimensional image from said reconstructed images on the plurality of said surfaces.

Optionally, the controller filters said rebinned data to maximize resolution of said three dimensional image. The rebinning of each of said detected X-ray onto a non-flat surface is achieved by collecting data from X-rays close to a surface with more than one sheet and wherein reconstruction on each surface is subsequently achieved by applying at least one of a two-dimensional weighted inverse Radon transform or an adapted two- dimensional reconstruction, to combined data from all sheets.

The three dimensional image is derived from a set of simultaneously solvable equations relating a plurality of reconstructed superimposed images, one on each of a plurality of multi-sheet surfaces, and a plurality of z-positions of sheets of each multi-sheet surface intersecting each point in a region being imaged. The set of simultaneously solvable equations is solved using at least one of a least squares sense, a minimization of a sum of absolute values of residuals, a minimization of any weighted norm of residuals, a minimization of any weighted norm of residuals wherein said weights are derived from a model of data errors, structured Total Least Norms, Iteratively Reweighted Least Squares and Iteratively Reweighted Norm approaches, or optimization methods including primal-dual methods, gradient methods, gradient projection methods, nonlinear reconstruction methods, with any type of regularization, penalty or constraints.

The controller initiates the generation of said three dimensional image after a threshold amount of detected X-ray data are obtained prior to obtaining data for the entire object. The plurality of X-ray sources are stationary. The controller uses said set of simultaneous equations incorporating the direction point spread functions in an axial direction to improve approximation with detected X-rays which were at least partially not on a rebinning surface. The controller is adapted to perform backprojection without filtering on multi-sheet surfaces; axial deconvolution; and subsequently filtering on each transaxial slice of a volume being imaged. The controller is adapted to correct sinogram data on a rebinning surface using at least one of an optical flow technique or any Partial Differential Equation technique.

The present invention also provides a method of generating a three dimensional image in a tomography system comprising comprises a plurality of X-ray or gamma ray sources and a plurality of detectors, which are offset from one other such that the detectors are not located directly opposite the sources, wherein the plurality of sources lie in a first plane and the plurality of detectors lie in a plurality of planes parallel to the first plane of sources, comprising the steps of: a) rebinning each of said detected X-rays or gamma rays onto a non-flat surface, b) performing a two dimensional reconstruction of said rebinned data on a non-flat surface, and c) generating said three dimensional image from said reconstructed images on the plurality of said surfaces.

Optionally, the method further comprises the step of filtering said rebinned data to maximize resolution of said three dimensional image. The step of rebinning of each of said detected X-ray onto a non-flat surface is achieved by collecting data from X-rays close to a surface with more than one sheet and wherein the reconstruction on each surface is subsequently achieved by applying at least one of a two-dimensional weighted inverse Radon transform or any adapted two- dimensional reconstruction, to combined data from all sheets.

The step of generating the three dimensional image is performed by solving a set of simultaneously solvable equations relating a plurality of reconstructed superimposed images, one on each of a plurality of multi-sheet surfaces, and a plurality of z-positions of sheets of each multi-sheet surface intersecting each point in a region being imaged. The set of simultaneously solvable equations is solved using at least one of a least squares, a minimization of a sum of absolute values of residuals, a minimization of any weighted norm of residuals, a minimization of any weighted norm of residuals where said weights are derived from a model of data errors, structured Total Least Norms, Iteratively Reweighted Least Squares and Iteratively Reweighted Norm approaches, optimization methods including primal-dual methods, gradient methods, gradient projection methods, nonlinear reconstruction methods, with any type of regularization, penalty or constraints.

The method uses said set of simultaneous equations incorporating the direction point spread functions in an axial direction to improve approximation with detected X-rays which were at least partially not on a rebinning surface. The method includes the steps of performing backprojection without filtering on multi-sheet surfaces; axial deconvolution; and subsequently filtering on each transaxial slice of a volume being imaged. The method of generating said three dimensional image is initiated after a threshold amount of detected X-ray data are obtained well before the data for the entire object has been acquired. The method further comprises the step of correcting sinogram data on a rebinning surface using at least one of an optical flow technique or any Partial Differential Equation technique.

These and other embodiments and aspects of the disclosed inventions will be further detailed in the Detailed Description read in light of the drawings.

## BRIEF DESCRIPTION OF THE DRAWINGS

These and other features and advantages of the present invention will be appreciated, as they become better understood by reference to the following detailed description when considered in connection with the accompanying drawings, wherein:

FIG. 1 is a graphical representation of a typical optimal rebinning surface with two sheets;
FIG. 2 is a graphical representation of an optimal single rebinning surface for an exemplary RTT geometry;
FIG. 3 is a graphical representation of the lower and upper bounds constricting the measurable rays, in an example where data is obtained using the RTT system;
FIG. 4A is a graphical representation of a standard truncated cone beam source; and
FIG. 4B is a graphical representation of an offset geometry system such as the RTT.

## DETAILED DESCRIPTION OF THE INVENTION

The present invention provides a method of reconstructing images from a cone beam tomographic sensor in which detectors are not located directly opposite radiation sources. In one embodiment, the present invention applies to X-ray computerized tomography. More generally, the present invention applies to methods of image reconstruction, wherein, radiation is assumed to propagate along straight lines through an attenuating medium. In an embodiment, the method of the present invention may be applied to image reconstruction in systems using gamma rays. The method of reconstructing images uses data from rays close to a multi-sheet surface which may have a conical singularity where the sheets of the surface meet or the sheets may cross along contours. This data is reconstructed in a manner similar to reconstruction of data from rays in a plane, by using a two dimensional reconstruction algorithm on a plurality of sheets. The volumetric image is recovered by solving a system of simultaneous equations, each of which expresses the superposition condition for all sheets of a multi-sheet surface. Such a system is solvable due to a plurality of sheets intersecting each voxel.

The present invention is directed towards multiple embodiments. The following disclosure is provided in order to enable a person having ordinary skill in the art to practice the invention. Language used in this specification should not be interpreted as a general disavowal of any one specific embodiment or used to limit the claims beyond the meaning of the terms used therein. The general principles defined herein may be applied to other embodiments and applications without departing from the scope of the invention. Also, the terminology and phraseology used is for the purpose of describing exemplary embodiments and should not be considered limiting. Thus, the present invention is to be accorded the widest scope encompassing numerous alternatives, modifications and equivalents consistent with the principles and features disclosed. For purpose of clarity, details relating to technical material that is known in the technical fields related to the invention have not been described in detail so as not to unnecessarily obscure the present invention.

The present invention employs a surface with a plurality of sheets, which in one embodiment intersect each other. Further, line data is approximated on the multi-sheet surface by the collected data. A two-dimensional image reconstruction is performed on data from all of the sheets together and then a system of linear equations is solved to recover the image at each point in the object. In X-ray tomography systems such as Real Time Tomography (RTT) system comprising multiple sources, the source trajectory is not limited to a helical path relative to an object being imaged. By varying a firing order of the sources and the rate of translation of the object relative to the source and detector array an effect equivalent to a source trajectory approximating a multi-threaded helix of variable pitch can be obtained. It should be noted that any other trajectory can be obtained depending upon the firing order of the sources and thus the invention is not limited to a multi-threaded helix. In one embodiment of the present invention, a complete set of sources is fired before any one source is fired for a second or subsequent time, and the object is translated only in one direction.

In one embodiment of the present invention, z represents a coordinate in an axial direction which is the direction of the translation of the object; x *and y* represent a coordinate system on a trans-axial plane which is a plane orthogonal to the direction of the translation of the object; and $\lambda$ represents a variable that is used to parameterize a curve which is monotonically increasing in z and passes through each source location in a tomography system such as RTT system in the order of their firing.

The following equation:

$$a(\lambda) = (a_1(\lambda), a_2(\lambda), a_3(\lambda)) \qquad (1)$$

represents a curve encircling the region of interest. In one embodiment, in a tomography system such as the RTT, it is a curve having a radius equal to the radius of a ring of sources. In an embodiment where the sources approximate a helical trajectory, $\lambda$ is proportional to the angular polar coordinate in the trans-axial plane.

In various embodiments, irrespective of the actual shape of the detector array, the rays through the source point $a(\lambda)$ are parameterized by Cartesian coordinates on a plane through the z-axis normal to $(a_1(\lambda), a_2(\lambda), 0)$. This plane is termed as a virtual detector plane and Cartesian coordinates $(u, v)$ are used on this plane. For each $\lambda$ and $u$, a ray given by a rebinning row function $v = V(\lambda, u)$, and a surface that is the graph of a function $\zeta(x, y)$, is chosen. Persons of ordinary skill in the art would know how to obtain an optimal surface $\zeta$ and rebinning function V.

A three dimensional image f(x,y,z) is reconstructed as a series of images on surfaces $\zeta_0(x, y)$, prescribed by the following function for multiple $\lambda_0$:

$$f_{\lambda_0}(x,y) = f(x, y, \zeta_{\lambda_0}(x, y)) \tag{2}$$

where: $x^2 + y^2 < R_{FOV}^2$; and $R_{FOV}$ represents a radius of the field of view.

Assuming a continuous source trajectory, a known algorithm for rebinning surfaces, and a function generation for helical cone beam computer tomography minimizes the following function:

$$Q(V_0, \zeta_0) = \int_{\lambda_0 - \pi/2 - \delta}^{\lambda_0 + \pi/2 + \delta} d\lambda \int_{-u_m}^{u_m} du \int_{l_0(u) - \Delta(u)}^{l_0(u) + \Delta(u)} dl \frac{p(u, \lambda)}{l} (\delta z(\lambda, u, l))^2 \tag{3}$$

where:

$$\delta z(\lambda, u, l) = h\lambda + lV_0(\lambda, u) - \zeta_0(X(\lambda, u, l), Y(\lambda, u, l)); \tag{4}$$

and p is a Parker weight.

Equation 3 may be solved with the following convergent iteration:

$$V_0(\lambda, u) = \frac{1}{2l_0(u)\Delta l(u)} \int_{l_0(u) - \Delta l(u)}^{l_0(u) + \Delta l(u)} dl (\zeta_0(X(\lambda, u, l), Y(\lambda, u, l)) - h\lambda) \tag{5}$$

$$\zeta_0(x, y) = \frac{1}{\int d\lambda p(U, \lambda)/L^2} \int_{\lambda_0 - \pi/2 - \delta}^{\lambda_0 + \pi/2 + \delta} d\lambda \frac{p(U(x, y, \lambda), \lambda)}{L(x, y, \lambda)^2} (h\lambda + V_0(U, \lambda)L) \tag{6}$$

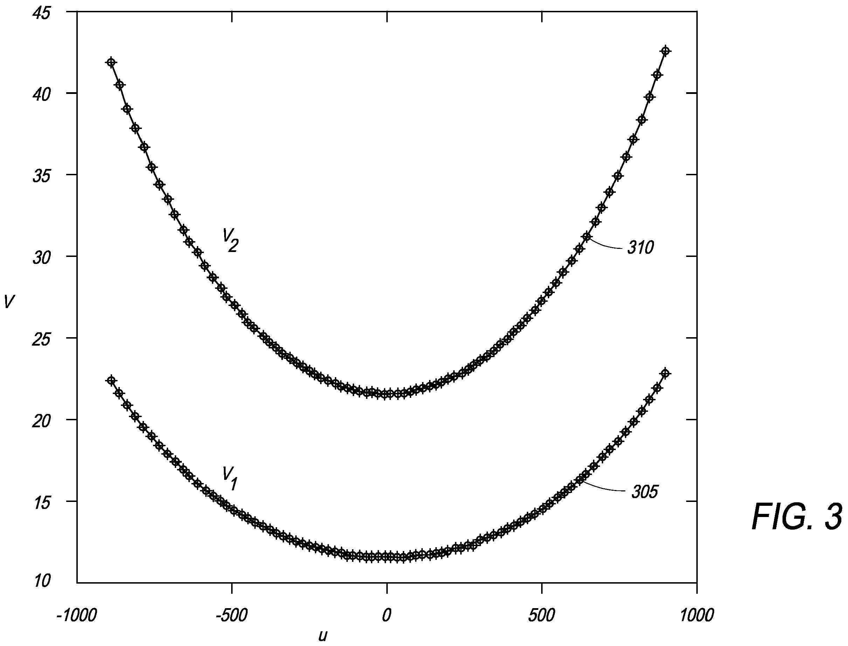

In a tomography system such as the RTT system, the location of the detector array relative to the source poses a constraint on the measurement of rays. When the detector array is projected on to a virtual detector plane the rays that can be measured are constrained by a lower bound $v_1(\lambda, u)$ and an upper bound $v_2(\lambda, u)$. FIG. 3 is a graphical representation of the lower and upper bounds 305 and 310, respectively, constricting the measurable rays.

In an embodiment of the present invention, constraints are accommodated on the detector for a system with a truncated or offset detector array $v_1(\lambda, u) \le V_0(\lambda, u) \le v_2(\lambda, u)$ by using Lagrange multipliers $\mu_1$ and $\mu_2$. In order to accommodate an arbitrary firing order the continuous source trajectory is replaced by a set of fired sources $S_A$, where $S_A$ contains sources with $\lambda \in [\lambda_0 - \pi/2 - \delta, \lambda_0 + \pi/2 + \delta]$ for each rebinning center $\lambda_0$.

$$Q(\zeta_0, V_0) = \sum_{\lambda \in S_A} \int_{-u_m}^{u_m} du \int_{l_0(u) - \Delta(u)}^{l_0(u) + \Delta(u)} dl w(\lambda, u, l) \left| \delta z(\lambda, u, l) \right|^q + \mu_1(v_1 - V_0) + \mu_2(V_0 - v_2) \tag{7}$$

where:

$$\delta z(\lambda,u,l) = z(\lambda) + lV_0(\lambda,u) - \zeta_0(X(\lambda,u,l),Y(\lambda,u,l)) \qquad (8)$$

where $z(\lambda)$ gives the z-translation of the source $\lambda$ at the moment it fires. The exponent q $\geq$ 1 determines the norm to be minimized or other measure for 0 < q < 1. In the simplest case q = 2 where the mean square axial deviation of rays from the rebinning surface the objective function is:

$$Q(V_0,\zeta_0) = \sum_{\lambda \in S_A} \int_{-u_m}^{u_m} du \int_{l_0(u)-\Delta(u)}^{l_0(u)+\Delta(u)} dl \frac{p(u,l)}{l}(\delta z(\lambda,u,l))^2 + \mu_1(v_1 - V_0) + \mu_2(V_0 - v_2) \qquad (9)$$

Equation (9) may be solved by using convergent iteration as follows:

$$\widetilde{V}_0(\lambda,u) = \frac{1}{2l_0(u)\Delta l(u)} \int_{l_0(u)-\Delta l(u)}^{l_0(u)+\Delta l(u)} dl(\zeta_0(X(\lambda,u,l),Y(\lambda,u,l) - z(\lambda)) \qquad (10)$$

$$V_0(\lambda,u) = \begin{cases} \widetilde{V}_0(\lambda,u) & v_1(\lambda,u) \leq \widetilde{V}_0(\lambda,u) \leq v_2(\lambda,u) \\ v_1(\lambda,u) & \widetilde{V}_0(\lambda,u) < v_1(\lambda,u) \\ v_2(\lambda,u) & \widetilde{V}_0(\lambda,u) > v_2(\lambda,u) \end{cases} \qquad (11)$$

$$\zeta_0(x,y) = \frac{1}{\sum_{s \in S_A} p(U,\lambda)/L^2} \sum_{\lambda \in S_A} \frac{p(U(x,y,\lambda),\lambda)}{L(x,y,\lambda)^2}(z(\lambda) + V_0(U,\lambda)L) \qquad (12)$$

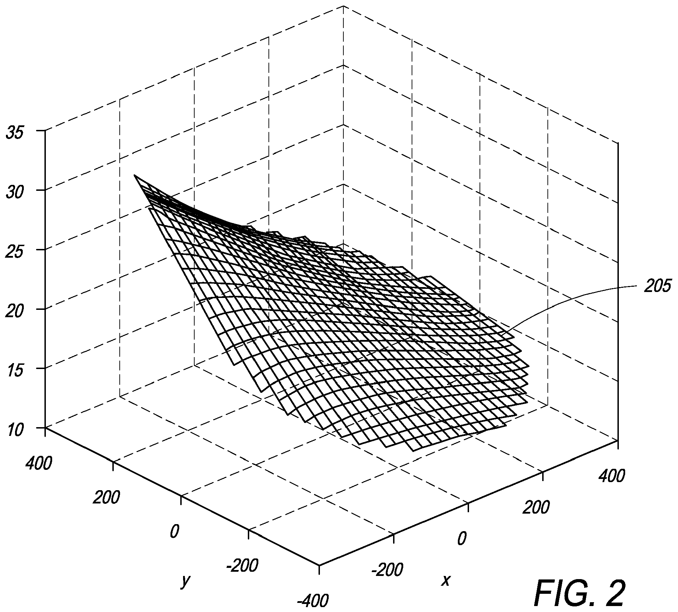

FIG. 2 is a graphical representation of an optimal single rebinning surface 205 for the RTT geometry.
In various embodiments of the present invention, a set of equations analogous to Equations (1), (2) and (7) through (12) are used for the construction of an optimal multi-sheet surface as well. For the purpose of illustration only, the procedure for a two sheet surface is described. In this case, the same cost functional is minimized and one rebinning function and two rebinning surfaces are obtained, here $S_A$ contains sources with $\lambda \in [\lambda_0 - \pi, \lambda_0 + \pi]$ for each rebinning center $\lambda_0$.

$$Q(\zeta_0,V_0) = \sum_{\lambda \in S_A} \int_{-u_m}^{u_m} du \int_{l_0(u)-\Delta l(u)}^{l_0(u)} dlw^s(\lambda,u,l)|\delta z^s(\lambda,u,l)|^p$$

$$+ \int_{l_0(u)}^{l_0(u)+\Delta l(u)} dlw^s(\lambda,u,l)|\delta z^s(\lambda,u,l)|^p \qquad (13)$$

$$+ \mu_1(v_1 - V_0^s) + \mu_2(V_0^s - v_2)$$

where

$$\delta z^s(\lambda,u,l) = z(\lambda) + lV_0(\lambda,u) - \zeta_0^s(X(\lambda,u,l),Y(\lambda,u,l)) \qquad s \in \{b,t\} \qquad (14)$$

and the two sheets of the surface are denoted by $\zeta_b(x, y)$, representing the bottom surface and $\zeta_t(x, y)$, representing the top surface.

Provided below is a derivation of a particular set of weights which fit the approximation that is being made in the method, but the principle holds for any set of positive weights. The case p = 2 is used because it yields the strictly convex objective function which has a unique global minimum which can be found by means of a globally convergent iteration. However, other choices of p are plausible e.g. p = 1 which would punish outliers less. In principle, once the solution of the least squares problem can be obtained any p $\geq$ 1 norm fit can be obtained by, for example, the iteratively reweighted least squares method or p > 0 by iteratively reweighted $L_1$ method.

In the particular case of p = 2, the square axial deviation reads

$$Q(\zeta_0, V_0) = \sum_{\lambda \in S_A} \int_{-u_m}^{u_m} du \int_{l_0(u)-\Delta(u)}^{l_0(u)} dl (\frac{1}{l} + \frac{1}{2l_o - l})(\delta^b z(\lambda, u, l))^2$$

$$+ \int_{l_0(u)}^{l_0(u)+\Delta(u)} dl (\frac{1}{l} + \frac{1}{2l_o - l})(\delta^t z(\lambda, u, l))^2 \qquad (15)$$

$$+ \mu_1(v_1 - V_0) + \mu_2(V_0 - v_2)$$

This can again be solved by the following globally convergent alternating iteration.

$$\tilde{V}_0(\lambda, u) = \frac{1}{4l_o(u)^2 \ln(\frac{l_0 + \Delta l}{l_0 - \Delta l}) - 4l_0(u)\Delta l(u)} \times$$

$$\left( \int_{l_0(u)-\Delta l(u)}^{l_0(u)} dl (1 + \frac{l}{2l_o - l})(\zeta_0^b(X(\lambda, u, l), Y(\lambda, u, l)) - z(\lambda)) + \int_{l_0(u)}^{l_0(u)+\Delta l(u)} dl (1 + \frac{l}{2l_o - l})(\zeta_0^t(X(\lambda, u, l), Y(\lambda, u, l)) - z(\lambda)) \right) \qquad (16)$$

$$V_0(\lambda, u) = \begin{cases} \tilde{V}_0(\lambda, u) & v_1(\lambda, u) \le \tilde{V}_0(\lambda, u) \le v_2(\lambda, u) \\ v_1(\lambda, u) & \tilde{V}_0(\lambda, u) < v_1(\lambda, u) \\ v_2(\lambda, u) & \tilde{V}_0(\lambda, u) > v_2(\lambda, u) \end{cases} \qquad (17)$$

$$\zeta_0^s(x, y) = \frac{1}{\sum_{\lambda \in S_A}(1/L^2 + 1/(L(2L_0 - L)))} \times \sum_{\lambda \in s_a}(\frac{1}{L^2} + \frac{1}{L(2L_0 - L)})(z(\lambda) + LV_0(\lambda, U)), \qquad (18)$$

where $L \le L_0$ for $s = b$ and $L \ge L_0$ for $s = t$.

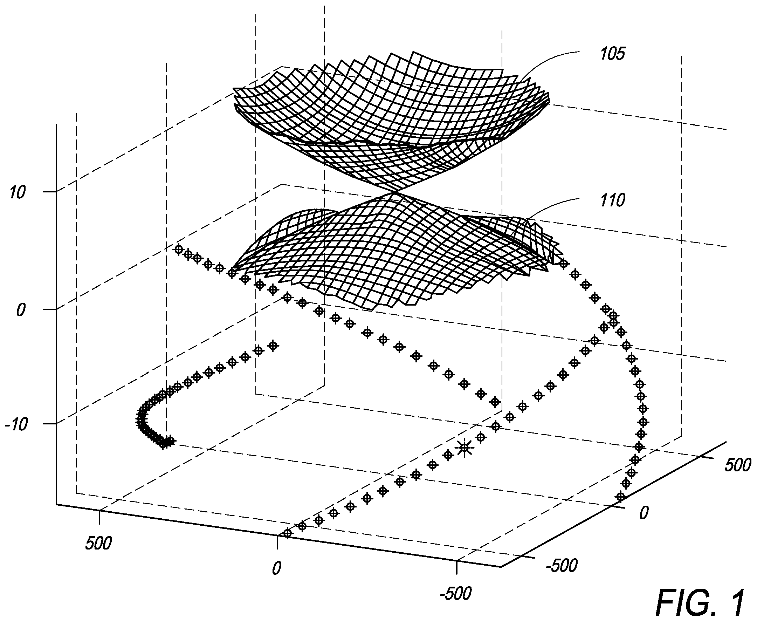

FIG. 1 is a graphical representation of a typical optimal rebinning surface with two sheets, 105 and 110.

With the two sheet surface $\zeta_0^s$, $s \in \{t,b\}$ on each sheet of the surface $\zeta_0^s$ we define the following 2D fan beam transform:

$$p_0^s(\lambda, u) = \sqrt{R^2 + u^2} \int_{l_0(u)-\Delta l(u)}^{l_0(u)+\Delta l(u)} dl f_{\zeta_0^s}(X(\lambda, u, l), Y(\lambda, u, l)). \qquad (19)$$

Then, the 2D fan beam transform on the entire surface $\zeta_0$ (including all its sheets) is the superposition of the fan beam transforms on all the individual sheets

$$p_0(\lambda, u) = \sum_s p_0^s(\lambda, u), \quad s \in \{t,b\} \qquad (20)$$

We also define the following mixed 2D fan beam transform on the multi-sheet surface $\zeta_0$

$$\tilde{g}_0(\lambda, u) = \tilde{g}_0^b(\lambda, u) + \tilde{g}_0^t(\lambda, u),$$

where

$$\widetilde{g}_0^b(\lambda,u) = \sqrt{R^2+u^2}\int_{l_0(u)-\Delta l(u)}^{l_0(u)} dl f_{\zeta_0^b}(X(\lambda,u,l),Y(\lambda,u,l))$$

$$\widetilde{g}_0^t(\lambda,u) = \sqrt{R^2+u^2}\int_{l_0(u)}^{l_0(u)+\Delta l(u)} dl f_{\zeta_0^t}(X(\lambda,u,l),Y(\lambda,u,l)) \qquad (21)$$

Note, that the first integral is taken over $\zeta_0^b$ and the second over $\zeta_0^t$. This mixed fan beam transform is a quantity which is the closest to the cone beam data as measured by the RTT. The idea of multi-sheet surface rebinning methods is to approximate $\widetilde{g}_0$ by the rebinned data $g_0$.

$$\widetilde{g}_0(\lambda,u) \approx g_0(\lambda,u), \qquad (22)$$

Where $g_0$ denotes the cone beam data which were rebinned to the surface $\zeta_0$ using the rebinning function $V_0$.

$$g_0(\lambda,u) = \sqrt{R^2+u^2}\int_{l_0(u)-\Delta l(u)}^{l_0(u)+\Delta l(u)} dl f(X(\lambda,u,l),Y(\lambda,u,l),z(\lambda)+lV_0(\lambda,u)) \qquad (23)$$

With analogous splitting

$$g_0(\lambda,u) = g_0^b(\lambda,u) + g_0^t(\lambda,u),$$

where

$$g_0^b(\lambda,u) = \sqrt{R^2+u^2}\int_{l_0(u)-\Delta l(u)}^{l_0(u)} dl f(X(\lambda,u,l),Y(\lambda,u,l),z(\lambda)+lV_0(\lambda,u))$$

$$g_0^t(\lambda,u) = \sqrt{R^2+u^2}\int_{l_0(u)}^{l_0(u)+\Delta l(u)} dl f(X(\lambda,u,l),Y(\lambda,u,l),z(\lambda)+lV_0(\lambda,u)) \qquad (24)$$

it holds

$$p_0^s(\lambda,u) = \widetilde{g}_0^s(\lambda,u) + \widetilde{g}_0^s(\lambda_c,u_c) \approx g_0^s(\lambda,u) + g_0^s(\lambda_c,u_c), \quad s \in \{b,t\} \qquad (25)$$

where ($\lambda_c$, $u_c$) denotes the ray along the same line as ($\lambda,u$) but traveling in the opposite direction. It therefore follows:

$$p_0(\lambda,u) = p_0^b(\lambda,u) + p_0^t(\lambda,u) = \widetilde{g}_0(\lambda,u) + \widetilde{g}_0(\lambda_c,u_c) \approx g_0(\lambda,u) + g_0(\lambda_c,u_c) \qquad (26)$$

This equation shows that both $p_0(\lambda,u)$ and $\widetilde{g}_0(\lambda, u) + \widetilde{g}_0(\lambda_c,u_c)$ describe the same 2D fan beam transform on the multi-sheet surface $\zeta_0$ (i.e., on all its sheets). Since we have a way to approximate the latter, we can approximate $p_0(\lambda,u)$ as well. Due to the linearity of the ray transform, $p_0$ is also the 2D fan beam transform of superposition of the objects on all sheets of the surface $\zeta_0$. This fact is then used to recover the volumetric density function f via deconvolution.

In an exemplary scenario, the constraint on the detector is such that only upward directed rays are detected. Hence, $a_3(\lambda) < v_1(\lambda, 0)$. The intersection of the ray with the region of interest is divided into two equal intervals; an interval closer to $a(\lambda)$ where the ray is close to the surface $\zeta_b$, and another interval where it is closer to $\zeta_t$.

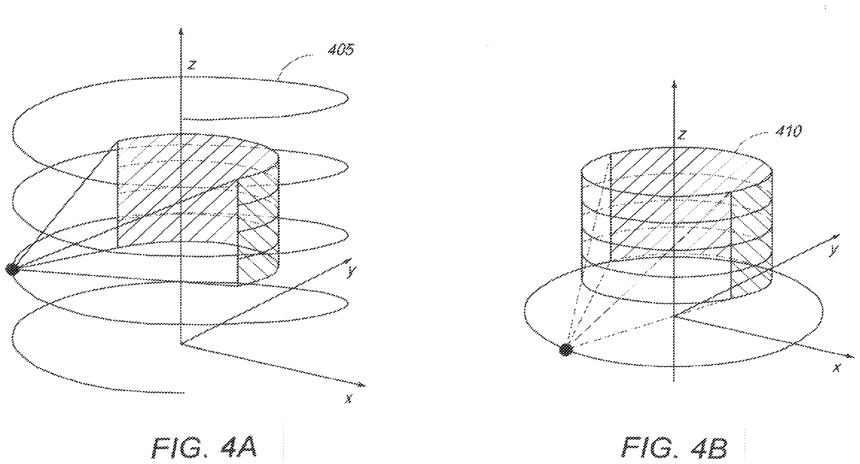

FIG.4A is a graphical representation of a standard truncated cone beam source 405 and FIG. 4B is a graphical representation of an offset geometry system 410, such as the RTT. In an embodiment of the present invention, the optimal choice of double-sheet surface that minimizes the total Q over both sheets is found to be in the shape roughly like a two pointed cone as illustrated in FIG.1. Each voxel lies on the intersection of some sheets for different multi-sheet surfaces. Each point value on the multi-sheet surface is a sum of point values on all sheets or a sum of the entire z-direction neighborhoods of the points on the sheets weighted with Point Spread Functions (PSF) of the sheets. This results in a sparse system of simultaneous equations to be solved for recovering the value of $f(x,y,z)$ at a specific voxel. In the simplest discretization, each row of this system of equations would have only two non-zero entries. Modelling PSF of the sheets and incorporating it into the matrix results in a slightly less sparse but more stable system. For conventional rebinning, known ultrahyperbolic equations can be used to improve the accuracy of approximation and this can also be applied to multi-sheet surface rebinning.

Hence, the present invention provides a tomography system and method in which the reconstruction of a three dimensional image is performed by collecting data from rays close to a surface with more than one sheet. A two-dimensional properly weighted inverse Radon transform is applied to the combined data from all sheets of one multi-sheet surface. This is repeated for a number of multi-sheet surfaces with rebinning centers chosen among fired sources. In one embodiment, the rebinning centers are equispaced in the z-direction, and their number is at least as large as the required z-resolution resulting in more than one sheet passing through each point in the region to be imaged. A system of simultaneous equations with a matrix, which one row involves information about where each sheet of one surface intersects the region to be imaged is then solved to produce a three dimensional image. The properties of this system, including independence of points in xy-plane, only local dependence on the z variable or possibility of precomputing many quantities mean that it can be solved efficiently.

The system is possibly ill-conditioned or right hand sides contain errors hence regularization is needed and a regularization parameter needs to be selected. Regularization methods that can be employed include quadratic penalty methods such as Tikhonov regularization, generalized Tikhonov regularization using a quadratic penalty applied to a weighted sum of derivatives, using any weighted norm of any order of derivatives including 0, the iterative regularization, total variation regularization, nonnegativity constraints, or using any type of sparsity constraints in different bases, including Curvelets or Wavelets. Possible methods for solution of the regularized system include a least squares sense, a minimization of a sum of absolute values of residuals, a minimization of any weighted norm of residuals, a minimization of any weighted norm of residuals where said weights are derived from a model of data errors, (structured) Total Least Norms, Iteratively Reweighted Least Squares and Iteratively Reweighted Norm approaches, and optimization methods, including primal-dual methods, gradient methods, gradient projection methods, nonlinear reconstruction methods. The regularization parameter can be chosen differently for different blocks of equations using methods including L-curve, Generalized Cross Validation and Unbiased Predictive Risk Estimation. Different grid sizes can be employed for different quantities at all stages resulting in efficiency increase or additional stability, examples include coarse grid matrix modeling leading to problems with multiple right hand sides or simultaneous regularization in the z-direction and within blocks in the *xy*-plane. Approximations leading to further symmetries may lead to even more memory and computationally efficient solutions.

It should be appreciated that the analytical methods described herein are performed by a controller, which has at least one processor executing a plurality of programmatic instructions embodying the analytical methods described herein. The instructions, along with the requisite data, are stored in a memory that is accessible to the processor in either a remote or local configuration. The input data is obtained from one or more detectors integrated into an X-ray scanning system.

While the exemplary embodiments of the present invention are described and illustrated herein, it will be appreciated that they are merely illustrative. It will be understood by those skilled in the art that various changes in form and detail may be made therein without departing from or offending the scope of the invention.

## Claims

1. A tomography system comprising a plurality of X-ray or gamma ray sources and a plurality of detectors, which are offset from one other such that the detectors are not located directly opposite the sources, wherein the plurality of sources lie in a first plane and the plurality of detectors lie in a plurality of planes parallel to the first plane of sources, further comprising a controller adapted to process X-rays or gamma rays detected by said plurality of detectors and generate a three dimensional image wherein said controller comprises a plurality of programmatic instructions that, when executed, a) rebin each of said detected X-rays or gamma rays onto a non-flat surface, achieved by collecting data from X-rays close to a surface with more than one sheet b) perform a two dimensional reconstruction of said rebinned data on a non-flat surface, and c) generate said three dimensional image from said reconstructed images on the plurality of said surfaces.

2. The system of claim 1 wherein said rebinning of each of said detected X-rays or gamma rays onto a non-flat surface is achieved by collecting data from X-rays or gamma rays close to a surface with more than one sheet and wherein reconstruction on each surface is subsequently achieved by applying at least one of a two-dimensional weighted inverse Radon transform or an adapted two- dimensional reconstruction, to combined data from all sheets.

3. The system of claim 2 wherein the three dimensional image is derived from a set of simultaneously solvable equations relating a plurality of reconstructed superimposed images, one on each of a plurality of multi-sheet surfaces, and a plurality of z-positions of sheets of each multi-sheet surface intersecting each point in a region being imaged.

4. The system of claim 3 wherein said set of simultaneously solvable equations is solved using at least one of a least squares sense, a minimization of a sum of absolute values of residuals, a minimization of any weighted norm of residuals, a minimization of any weighted norm of residuals wherein said weights are derived from a model of data errors, structured Total Least Norms, Iteratively Reweighted Least Squares and Iteratively Reweighted Norm approaches, or optimization methods including primal-dual methods, gradient methods, gradient projection methods, nonlinear reconstruction methods, with any type of regularization, penalty or constraints.

5. The system of claim 1 wherein the controller initiates the generation of said three dimensional image after a threshold amount of detected X-ray or gamma ray data are obtained prior to obtaining data for the entire object; or
wherein the plurality of X-ray or gamma ray sources are stationary; or
wherein said controller is adapted to correct sinogram data on a rebinning surface using at least one of an optical flow technique or any Partial Differential Equation technique.

6. The system of claim 3 wherein said controller uses said set of simultaneous equations incorporating the direction point spread functions in an axial direction to improve approximation with detected X-rays or gamma rays which were at least partially not on a rebinning surface.

7. The system of claim 2 wherein said controller is adapted to perform backprojection without filtering on multi-sheet surfaces; axial deconvolution; and subsequently filtering on each transaxial slice of a volume being imaged.

8. A method of generating a three dimensional image in a tomography system comprising a plurality of X-ray or gamma ray sources and a plurality of detectors, which are offset from one other such that the detectors are not located directly opposite the sources, wherein the plurality of sources lie in a first plane and the plurality of detectors lie in a plurality of planes parallel to the first plane of sources, comprising the steps of: a) rebinning each of said detected X-rays or gamma rays onto a non-flat surface, achieved by collecting data from X-rays close to a surface with more than one sheet b) performing a two dimensional reconstruction of said rebinned data on a non-flat surface, and c) generating said three dimensional image from said reconstructed images on the plurality of said surfaces.

9. The method of claim 8 wherein the step of rebinning of each of said detected X-rays or gamma rays onto a non-flat surface is achieved by collecting data from X-rays or gamma rays close to a surface with more than one sheet and wherein the reconstruction on each surface is subsequently achieved by applying at least one of a two-dimensional weighted inverse Radon transform or any adapted two- dimensional reconstruction, to combined data from all sheets.

10. The method of claim 9 wherein the step of generating the three dimensional image is performed by solving a set of simultaneously solvable equations relating a plurality of reconstructed superimposed images, one on each of a plurality of multi-sheet surfaces, and a plurality of z-positions of sheets of each multi-sheet surface intersecting each point in a region being imaged.

11. The method of claim 10 wherein said set of simultaneously solvable equations is solved using at least one of a least squares, a minimization of a sum of absolute values of residuals, a minimization of any weighted norm of residuals, a minimization of any weighted norm of residuals where said weights are derived from a model of data errors, structured Total Least Norms, Iteratively Reweighted Least Squares and Iteratively Reweighted Norm approaches, optimization methods including primal-dual methods, gradient methods, gradient projection methods, nonlinear reconstruction methods, with any type of regularization, penalty or constraints.

12. The method of claim 10, which uses said set of simultaneous equations incorporating the direction point spread functions in an axial direction to improve approximation with detected X-rays or gamma rays which were at least partially not on a rebinning surface.

13. The method of claim 9, wherein said method includes the steps of performing backprojection without filtering on multi-sheet surfaces; axial deconvolution; and subsequently filtering on each transaxial slice of a volume being imaged.

14. The method of claim 8 wherein the method of generating said three dimensional image is initiated after a threshold amount of detected X-ray or gamma ray data are obtained well before the data for the entire object has been acquired.

**15.** The method of claim 8 further comprising the step of correcting sinogram data on a rebinning surface using at least one of an optical flow technique or any Partial Differential Equation technique.

## Patentansprüche

**1.** Tomografiesystem, umfassend eine Vielzahl von Röntgenstrahlen- oder Gammastrahlenquellen und eine Vielzahl von Detektoren, die voneinander versetzt sind, sodass sich die Detektoren nicht direkt gegenüber den Quellen befinden, wobei die Vielzahl von Quellen in einer ersten Ebene liegt und die Vielzahl von Detektoren in einer Vielzahl von Ebenen parallel zu der ersten Ebene von Quellen liegt, ferner umfassend eine Steuerung, die dazu angepasst ist, Röntgenstrahlen oder Gammastrahlen zu verarbeiten, die von der Vielzahl von Detektoren erfasst wurden, und ein dreidimensionales Bild zu erzeugen, wobei die Steuerung eine Vielzahl von programmatischen Anweisungen umfasst, die, wenn ausgeführt, a) jeden der erfassten Röntgenstrahlen oder Gammastrahlen auf eine nicht ebene Oberfläche rebinnen, die durch Sammeln von Daten von Röntgenstrahlen nahe einer Oberfläche mit mehr als einer Scheibe erhalten wurden, b) eine zweidimensionale Rekonstruktion der gerebinnten Daten auf einer nicht ebenen Oberfläche durchführen und c) das dreidimensionale Bild aus den rekonstruierten Bildern auf der Vielzahl der Oberflächen erzeugen.

**2.** System nach Anspruch 1, wobei das Rebinning von jedem der erfassten Röntgenstrahlen oder Gammastrahlen auf eine nicht ebene Oberfläche erreicht wird durch Sammeln von Daten von Röntgenstrahlen oder Gammastrahlen nahe einer Oberfläche mit mehr als einer Scheibe und wobei die Rekonstruktion auf jeder Oberfläche anschließend erreicht wird durch Anwenden von mindestens einer von einer zweidimensionalen gewichteten umgekehrten Radon-Transformation oder einer angepassten zweidimensionalen Rekonstruktion auf kombinierte Daten von allen Scheiben.

**3.** System nach Anspruch 2, wobei das dreidimensionale Bild aus einem Satz von gleichzeitig lösbaren Gleichungen bezogen auf eine Vielzahl von rekonstruierten Überlagerungsbildern abgeleitet wird, wobei eine auf jeder einer Vielzahl von Mehrfachscheiben-Oberflächen, und eine Vielzahl von Z-Positionen von Scheiben von jeder Mehrfachscheiben-Oberfläche, die jeden Punkt in einem Bereich schneidet, abgebildet wird.

**4.** System nach Anspruch 3, wobei der Satz aus gleichzeitig lösbaren Gleichungen gelöst wird mithilfe von mindestens einer von einer Methode der kleinsten Quadrate, einer Minimierung einer Summe aus absoluten Restwerten, einer Minimierung von einer gewichteten Norm von Resten, einer Minimierung von einer gewichteten Norm von Resten, wobei die Gewichte aus einem Modell von Datenfehlern, einer strukturierten Norm der Methode der kleinsten Quadrate, iterativ neugewichteten kleinsten Quadraten und iterativ neugewichteten Normansätzen oder Optimierungsverfahren abgeleitet werden, die primal-duale Verfahren, Gradientenverfahren, Gradientenprojektionsverfahren, nichtlineare Rekonstruktionsverfahren mit einer beliebigen Art von Regularisierung, Strafe oder Beschränkungen umfassen.

**5.** System nach Anspruch 1, wobei die Steuerung die Erzeugung des dreidimensionalen Bildes initiiert, nachdem vor dem Erhalten von Daten für das gesamte Objekt eine Schwellenwertmenge von erfassten Röntgenstrahlen- oder Gammastrahlendaten erhalten wurde; oder
wobei die Vielzahl von Röntgenstrahlen- oder Gammastrahlenquellen ortsfest ist; oder
wobei die Steuerung dazu angepasst ist, Sinogrammdaten auf einer Rebinning-Oberfläche mithilfe von mindestens einer von einer optischen Flusstechnik oder einer beliebigen partiellen Differentialgleichungstechnik zu korrigieren.

**6.** System nach Anspruch 3, wobei die Steuerung den Satz gleichzeitiger Gleichungen, die die Richtungspunktausbreitungsfunktionen in einer Axialrichtung verwendet, um die Näherung mit erfassten Röntgenstrahlen oder Gammastrahlen zu verbessern, die mindestens teilweise nicht auf einer Rebinning-Oberfläche waren.

**7.** System nach Anspruch 2, wobei die Steuerung dazu angepasst ist, eine Rückprojektion ohne Filterung von Mehrfachscheiben-Oberflächen, axiale Dekonvolution und anschließendes Filtern jeder transaxialen Scheibe eines Volumens, das abgebildet wird, durchzuführen.

**8.** Verfahren zum Erzeugen eines dreidimensionalen Bildes in einem Tomografiesystem, umfassend eine Vielzahl von Röntgenstrahlen- oder Gammastrahlenquellen und eine Vielzahl von Detektoren, die voneinander versetzt sind, sodass sich die Detektoren nicht direkt gegenüber den Quellen befinden, wobei die Vielzahl von Quellen in einer ersten Ebene liegt und die Vielzahl von Detektoren in einer Vielzahl von Ebenen parallel zu der ersten Ebene von Quellen liegt, umfassend die Schritte: a) Rebinning eines jeden der erfassten Röntgenstrahlen oder Gammastrahlen auf eine nicht ebene Oberfläche, die durch Sammeln von Daten von Röntgenstrahlen nahe einer Oberfläche mit mehr als einer Scheibe erhalten wurden, b) Durchführen einer zweidimensionalen Rekonstruktion der gerebinnten Daten auf einer nicht ebenen Oberfläche und c) Erzeugen des dreidimensionalen Bildes aus den rekonstruierten Bildern auf der Vielzahl der Oberflächen.

9. Verfahren nach Anspruch 8, wobei der Schritt des Rebinnings von jedem der erfassten Röntgenstrahlen oder Gammastrahlen auf eine nicht ebene Oberfläche erreicht wird durch Sammeln von Daten von Röntgenstrahlen oder Gammastrahlen nahe einer Oberfläche mit mehr als einer Scheibe und wobei die Rekonstruktion auf jeder Oberfläche anschließend erreicht wird durch Anwenden von mindestens einer von einer zweidimensionalen gewichteten umgekehrten Radon-Transformation oder einer beliebigen angepassten zweidimensionalen Rekonstruktion auf kombinierte Daten von allen Scheiben.

10. Verfahren nach Anspruch 9, wobei der Schritt des Erzeugens des dreidimensionalen Bildes durchgeführt wird durch Lösen eines Satzes aus gleichzeitig lösbaren Gleichungen bezogen auf eine Vielzahl von rekonstruierten Überlagerungsbildern, wobei eine auf jeder einer Vielzahl von Mehrfachscheiben-Oberflächen und einer Vielzahl von Z-Positionen von Scheiben von jeder Mehrfachscheiben-Oberfläche, die jeden Punkt in einem Bereich schneidet, abgebildet wird.

11. Verfahren nach Anspruch 10, wobei der Satz aus gleichzeitig lösbaren Gleichungen gelöst wird mithilfe von mindestens einer von einer kleinsten Quadrate, einer Minimierung einer Summe aus absoluten Restwerten, einer Minimierung von einer gewichteten Norm von Resten, einer Minimierung von einer gewichteten Norm von Resten, wobei die Gewichte aus einem Modell von Datenfehlern, einer strukturierten Norm der Methode der kleinsten Quadrate, iterativ neugewichteten kleinsten Quadraten und iterativ neugewichteten Normansätzen, Optimierungsverfahren abgeleitet werden, die primal-duale Verfahren, Gradientenverfahren, Gradientenprojektionsverfahren, nichtlineare Rekonstruktionsverfahren mit einer beliebigen Art von Regularisierung, Strafe oder Beschränkungen umfassen.

12. System nach Anspruch 10, das den Satz gleichzeitiger Gleichungen, die die Richtungspunktausbreitungsfunktionen in einer Axialrichtung integriert, verwendet, um die Näherung mit erfassten Röntgenstrahlen oder Gammastrahlen zu verbessern, die mindestens teilweise nicht auf einer Rebinning-Oberfläche waren.

13. Verfahren nach Anspruch 9, wobei das Verfahren die Schritte des Durchführens einer Rückprojektion ohne Filterung von Mehrfachscheiben-Oberflächen, axialer Dekonvolution und anschließender Filterung jeder transaxialen Scheibe eines Volumens, das abgebildet wird, umfasst.

14. Verfahren nach Anspruch 8, wobei das Verfahren des Erzeugens des dreidimensionalen Bildes initiiert wird, nachdem eine Schwellenwertmenge der erfassten Röntgenstrahlen- oder Gammastrahlendaten erhalten wurde, lange bevor die Daten für das gesamte Objekt erfasst wurden.

15. Verfahren nach Anspruch 8, ferner umfassend den Schritt des Korrigierens von Sinogrammdaten auf einer Rebinning-Oberfläche mithilfe von mindestens einer von einer optischen Flusstechnik oder einer beliebigen partiellen Differentialgleichungstechnik.

**Revendications**

1. Système tomographique comprenant une pluralité de sources de rayons X ou de rayons gamma et une pluralité de détecteurs, qui sont décalés les uns des autres de telle sorte que les détecteurs ne sont pas situés juste en face des sources, la pluralité de sources se situant dans un premier plan et la pluralité de détecteurs se situant dans une pluralité de plans parallèles au premier plan de sources, comprenant en outre un contrôleur adapté pour traiter des rayons X ou rayons gamma détectés par ladite pluralité de détecteurs et générer une image tridimensionnelle, ledit contrôleur comprenant une pluralité d'instructions programmatiques qui, lorsqu'elles sont exécutées, a) réarrangent chacun desdits rayons X ou rayons gamma détectés sur une surface non plate, réalisé en collectant des données issues de rayons X proches d'une surface avec plus d'une feuille, b) réalisent une reconstruction bidimensionnelle desdites données réarrangées sur une surface non plate, et c) génèrent ladite image tridimensionnelle à partir desdites images reconstruites sur la pluralité desdites surfaces.

2. Système de la revendication 1 dans lequel ledit réarrangement de chacun desdits rayons X ou rayons gamma détectés sur une surface non plate est réalisé en collectant des données issues de rayons X ou de rayons gamma proches d'une surface avec plus d'une feuille et dans lequel une reconstruction sur chaque surface est ensuite réalisée en appliquant une transformée de Radon inverse pondérée bidimensionnelle et/ou une reconstruction bidimensionnelle adaptée, pour combiner des données issues de toutes les feuilles.

3. Système de la revendication 2 dans lequel l'image tridimensionnelle est dérivée d'un ensemble d'équations résolubles simultanément associant une pluralité d'images reconstruites superposées, une sur chacune d'une pluralité de surfaces multifeuilles, et une pluralité de positions en z de feuilles de chaque surface multifeuille coupant chaque point dans une région en train d'être imagée.

4. Système de la revendication 3 dans lequel ledit ensemble d'équations résolubles simultanément est résolu en utilisant au moins une technique parmi une détection par moindres carrés, une minimisation d'une somme de valeurs absolues de résidus, une minimisation de n'importe quelle norme pondérée de résidus, une minimisation de n'importe quelle norme pondérée de résidus dans laquelle lesdits poids sont dérivés d'un modèle d'erreurs de données, des approches de normes de moindres carrés totales structurées, de moindres carrés itérativement repondérés et de norme itérativement repondérée, ou des procédés d'optimisation incluant des procédés primal-dual, des procédés de gradient, des procédés de projection de gradient, des procédés de reconstruction non linéaire, avec n'importe quel type de régularisation, pénalité ou contraintes.

5. Système de la revendication 1 dans lequel le contrôleur initie la génération de ladite image tridimensionnelle après qu'une quantité seuil de données de rayons X ou de rayons gamma détectées a été obtenue avant d'obtenir des données pour l'objet entier ; ou
dans lequel la pluralité de sources de rayons X ou de rayons gamma sont stationnaires ; ou
dans lequel ledit contrôleur est adapté pour corriger des données de sinogramme sur une surface de réarrangement en utilisant une technique de flux optique et/ou une technique d'équation différentielle partielle.

6. Système de la revendication 3 dans lequel ledit contrôleur utilise ledit ensemble d'équations simultanées incorporant les fonctions d'étalement de points directionnels dans une direction axiale pour améliorer une approximation avec des rayons X ou rayons gamma détectés qui n'étaient au moins partiellement pas sur une surface de réarrangement.

7. Système de la revendication 2 dans lequel ledit contrôleur est adapté pour réaliser une rétroprojection sans filtrage sur des surfaces multifeuilles ; une déconvolution axiale ; et un filtrage ultérieur sur chaque tranche transaxiale d'un volume en train d'être imagé.

8. Procédé de génération d'une image tridimensionnelle dans un système tomographique comprenant une pluralité de sources de rayons X ou de rayons gamma et une pluralité de détecteurs, qui sont décalés les uns des autres de telle sorte que les détecteurs ne sont pas situés juste en face des sources, la pluralité de sources se situant dans un premier plan et la pluralité de détecteurs se situant dans une pluralité de plans parallèles au premier plan de sources, comprenant les étapes de : a) réarrangement de chacun desdits rayons X ou rayons gamma détectés sur une surface non plate, réalisé en collectant des données issues de rayons X proches d'une surface avec plus d'une feuille, b) réalisation d'une reconstruction bidimensionnelle desdites données réarrangées sur une surface non plate, et c) génération de ladite image tridimensionnelle à partir desdites images reconstruites sur la pluralité desdites surfaces.

9. Procédé de la revendication 8 dans lequel l'étape de réarrangement de chacun desdits rayons X ou rayons gamma détectés sur une surface non plate est réalisée en collectant des données issues de rayons X ou de rayons gamma proches d'une surface avec plus d'une feuille et dans lequel la reconstruction sur chaque surface est ensuite réalisée en appliquant une transformée de Radon inverse pondérée bidimensionnelle et/ou n'importe quelle reconstruction bidimensionnelle adaptée, à des données combinées issues de toutes les feuilles.

10. Procédé de la revendication 9 dans lequel l'étape de génération de l'image tridimensionnelle est réalisée en résolvant un ensemble d'équations résolubles simultanément associant une pluralité d'images reconstruites superposées, une sur chacune d'une pluralité de surfaces multifeuilles, et une pluralité de positions en z de feuilles de chaque surface multifeuille coupant chaque point dans une région en train d'être imagée.

**11.** Procédé de la revendication 10 dans lequel ledit ensemble d'équations résolubles simultanément est résolu en utilisant au moins une technique parmi une moindres carrés, une minimisation d'une somme de valeurs absolues de résidus, une minimisation de n'importe quelle norme pondérée de résidus, une minimisation de n'importe quelle norme pondérée de résidus dans laquelle lesdits poids sont dérivés d'un modèle d'erreurs de données, des approches de normes de moindres carrés totales structurées, de moindres carrés itérativement repondérés et de norme itérativement repondérée, ou des procédés d'optimisation incluant des procédés primal-dual, des procédés de gradient, des procédés de projection de gradient, des procédés de reconstruction non linéaire, avec n'importe quel type de régularisation, pénalité ou contraintes.

**12.** Procédé de la revendication 10, qui utilise ledit ensemble d'équations simultanées incorporant les fonctions d'étalement de points directionnels dans une direction axiale pour améliorer une approximation avec des rayons X ou rayons gamma détectés qui n'étaient au moins partiellement pas sur une surface de réarrangement.

**13.** Procédé de la revendication 9, ledit procédé comportant les étapes de réalisation d'une rétroprojection sans filtrage sur des surfaces multifeuilles ; déconvolution axiale ; et filtrage ultérieur sur chaque tranche transaxiale d'un volume en train d'être imagé.

**14.** Procédé de la revendication 8, le procédé de génération de ladite image tridimensionnelle étant initié après qu'une quantité seuil de données de rayons X ou de rayons gamma détectées a été obtenue bien avant que les données pour l'objet entier soient acquises.

**15.** Procédé de la revendication 8 comprenant en outre l'étape de correction de données de sinogramme sur une surface de réarrangement au moyen d'une technique de flux optique et/ou de n'importe quelle technique d'équation différentielle partielle.

FIG. 1

EP 2 453 798 B1

FIG. 2

*FIG. 3*

1

x

y

z

*FIG. 4B*

*FIG. 4A*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080049891 A1
- US 6240157 B1
- US 6411670 B1